# EUROPEAN PATENT APPLICATION

(11) **EP 2 942 058 A1**
(43) Date of publication of application: **11.11.2015**
(21) Application number: 15166952.0
(22) Date of filing: 08.05.2015
(51) Int. Cl.: A61K 31/5377, A61K 45/06, A61K 9/20, A61K 9/24, A61K 9/00, A61K 31/426, A61P 1/04, A61P 7/02

(54) **Pharmaceutical combinations of rivaroxaban and H2-receptor antagonists**

(30) Priority: 09.05.2014 TR 201405225; 10.09.2014 TR 201410626; 15.09.2014 TR 201410832
(71) Applicant: Sanovel Ilac Sanayi ve Ticaret A.S., 34460 Istanbul (TR)
(72) Inventor: Türkylmaz, Ali, 34460 Istanbul (TR); Yelken, Gülay, 34460 Istanbul (TR); Güner, Dicle, 34460 Istanbul (TR)
(74) Representative: Sevinç, Erkan

(57) **Abstract**

This invention is a novel pharmaceutical composition comprising rivaroxaban or a pharmaceutically acceptable salt thereof in combination with a H2-receptor antagonist for use in the anticoagulant treatment with preventing or reducing the risk of a gastrointestinal disorder.

## Description

### Field of Invention

This invention is a novel pharmaceutical composition comprising rivaroxaban or a pharmaceutically acceptable salt in a combination with a H2-receptor antagonist (H2RA) and at least one pharmaceutically acceptable excipient.

More specifically, this invention relates to pharmaceutical composition comprising rivaroxaban or a pharmaceutically acceptable salt thereof in combination with a H2-receptor antagonist (H2RA) for use in the anticoagulant treatment with preventing or reducing the risk of a gastrointestinal disorder administrated by oral, parenteral, intramuscular or topical route.

### Background of Invention

Anticoagulant drugs target various procoagulant factors in the coagulation pathway. Presently there are numerous anticoagulant drugs which are widely available. One of them is direct factor Xa inhibitors which plays a central role in the cascade of blood coagulation by inhibiting factor Xa directly. In an exemplary embodiment, the Direct factor Xa inhibitors is a member selected from rivaroxaban, apixaban, edoxaban, betrixaban, TAK-442, darexaban, and pro-drug s thereof.

Rivaroxaban (Formula I), which is already known from WO0147919, is an anticoagulant and the first orally active direct factor Xa inhibitor. Rivaroxaban is used to treat deep vein thrombosis (DVT) and pulmonary embolism (PE). It is also used to help prevent strokes or serious blood clots in people who have atrial fibrillation. 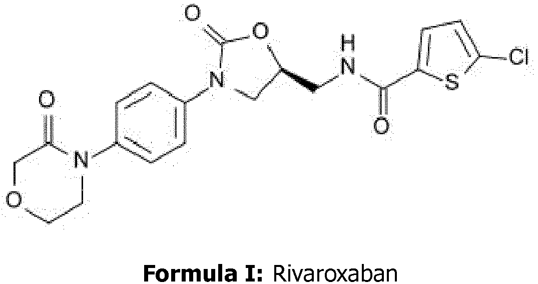 Rivaroxaban , which is commercially available under the trade name Xarelto®, is disclosed in US7157456 (B2), US7585860 (B2), US7592339 (B2).

However, rivaroxaban is associated with side effects. The administration of anticoagulant agents, such as rivaroxaban, have been associated with gastrointestinal disorders such as ulcers and gastrointestinal bleeding. In addition, the administration of rivaroxaban , may make some patients more susceptible to the ulcerogenic effects of ulcerogenic stimuli. It appears that a major factor contributing to the development of these gastrointestinal disorders is the presence of acid in the stomach and upper small intestine. While the mechanisms associated with ulcers and gastrointestinal bleeding are not entirely known, there are many causes of ulcers, including stress, alcohol irritation, Helicobacter pylori infection, and the side effects of non-steroid antiinflammatory drugs. Patients in need of long term anticoagulant drug therapy often may interrupt or not receive such therapy due to gastrointestinal disorders, and as a result, patients are deprived of beneficial anticoagulant drug therapy. There is a need for oral pharmaceutical combination formulations to reduce or eliminate the gastrointestinal disorders associated with use of anticoagulant drugs.

H2-receptor antagonists (H2RA) are competitive, reversible inhibitors of the action of histamine at the histamine H2-receptors found in gastric cells. This results in decreased gastric acid secretion and gastric volume, and reduced hydrogen ion concentration. The acid labile compounds are useful for inhibiting gastric acid secretion.

Famotidine is a histamine H2-receptor antagonist that inhibits stomach acid production. Chemical structure of famotidine (3-[[2-(diaminomethylideneamino)-1,3-thiazol-4-yl]methylsulfanyl]-N'-sulfamoylpropanimidamide) is illustrated with Formula II given below.

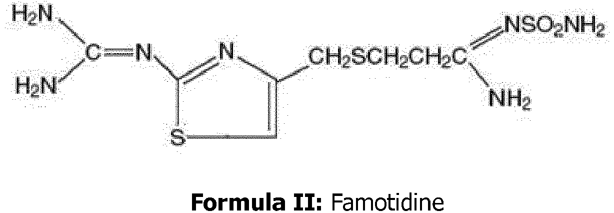

In prior art, US Patent Numbers US4362736 discloses the compound of famotidine. US Patent Numbers US4283408 discloses the medical composition containing famotidine as a gastric acid secretion inhibitor with pharmaceutically acceptable carriers or diluents. Famotidine is commercially available under the trade names PEPCIDINE and PEPCID®.

Nizatidine is a histamine H2-receptor antagonist with low toxicity that inhibits stomach acid production and gastric acid secretion. Chemical structure of nizatidine (N-(2-[(2-[(dimethylamino)methyl]thiazol-4-yl)methylthio]ethyl)-N-methyl-2-nitroethene-1,1-diamine) is illustrated with Formula III given below.

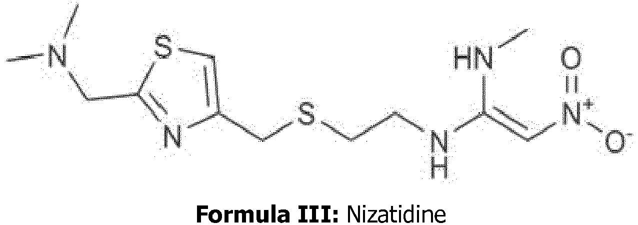

In prior art, US Patent Numbers US4777260 discloses the process for preparing a novel intermediate for nizatidine. US Patent Numbers US4375547 discloses the compound of nizatidine for inhibiting gastric acid secretion in mammals. Nizatidine is commercially available under the trade names TAZAC and AXID®.

Ranitidine is a histamine H2-receptor antagonist that inhibits stomach acid production. Chemical structure of ranitidine (N-(2-[(5-[(dimethylamino)methyl]furan-2-yl)methylthio]ethyl)-N'-methyl-2-nitroethene-1,1-diamine) is illustrated with Formula IV given below.

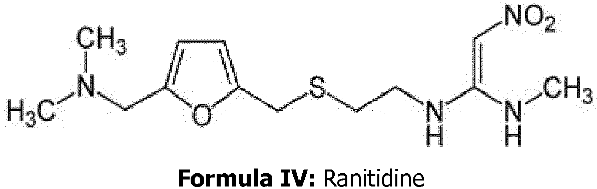

In prior art, US Patent Numbers US4128658 discloses the compound of ranitidine. Additionally ranitidine brings about an improvement of method of treating a condition mediated through histamine H2-receptors, especially for peptic ulceration. US Patent Numbers US5008256 discloses the compound of ranitidine bismuth citrate for treating or preventing gastrointestinal disorders. The bismuth citrate of ranitidine is commercially available under the trade name PYLORID®. EP Patent Numbers EP0626381 discloses a process for preparing form 1 ranitidine hydrochloride. The hydrochloride salt of ranitidine is commercially available under the trade name ZANTAC®.

As described above, the major factor contributing to the development of gastrointestinal disorders is the presence of acid in the stomach and upper small intestine. Therefore, the main object of the invention is administering to a patient an oral dosage form including a combination of an anticoagulant agent and a H2-receptor antagonist (H2RA) which can reduce a major factor contributing to the development of gastrointestinal disorder in patients. According to the main object, the pharmaceutical compositions of this invention is for the prevention gastrointestinal tract from side effects associated with anticoagulant therapy and provide greater anticoagulant effect with the results in a lower incidence of side effects. According to prior inventions rivaroxaban in combination with H2-receptor antagonists (H2RA) in particular famotidine or nizatidine or ranitidine have an additive preventive effect in relief of gastrointestinal disorder and provide greater anticoagulant effect with the results in a lower incidence of side effects.

### Detailed description of the invention

The present invention relates to a pharmaceutical composition comprising rivaroxaban in a combination with a H2-receptor antagonist (H2RA) or a pharmaceutically acceptable salt, solvate, hydrate, enantiomer, pro-drug or polymorph thereof and at least one pharmaceutically acceptable excipient

The main object of the present invention is to provide new pharmaceutical compositions comprising a specific direct factor Xa inhibitor such as rivaroxaban in combination with H2-receptor antagonist (H2RA) selected from the group comprising bisfentidine, burimamide, cimetidine, dalcotidine, donetidine, ebrotidine, etintidine, famotidine, lafutidine, lamtidine, lavoltidine , loxtidine, lupitidine, metiamide, mifentidine, niperotidine, nizatidine, osutidine, oxmetidine, pibutidine, quisultazine, quisultidine, ramixotidine, ranitidine, ranitidine bismuth citrate, ranitidine hydrochloride, roxatidine, sufotidine, tiotidine, tuvatidine, venritidine, xaltidine or other derivatives or a pharmaceutically acceptable salt, solvate, hydrate, enantiomer, prodrug or polymorph thereof as effective components which overcomes the above described problems in prior art and have additive advantages over them.

According to the formulation of the present invention it is desired to provide a dosage form comprising in combination a therapeutically effective amount of an anticoagulant agent, specifically rivaroxaban and a H2-receptor antagonist (H2RA) specifically famotidine or nizatidine or ranitidine which overcomes above described problems. The main challenges when combining those molecules in the same pharmaceutical form are:
(a) to guarantee the physico-chemical compatibility between those different active ingredients and/or between the active ingredients and the excipients used; and
(b) to insure the pharmaceutical compatibility between those active ingredients regarding their stability characteristics. When rivaroxaban is combined with H2RA specifically ranitidine, famotidine or nizatidine in order to minimize or prevent the side effects of the rivaroxaban, these two drug substances must be released, dissolved and absorbed harmoniously. For the efficiency of the formulation, an easy dissolution of both drug substances at a desired time is an important factor. In order to minimize or prevent the side effects of rivaroxaban; it is very important for the molecule, in which the molecule is used in combination with the H2RAs to release both drug substances at the desired time.

The product in this invention is used for protecting the gastrointestinal tract from side effects associated with anticoagulant therapy using the oral dosage forms described herein. The invention also relates to protect the gastrointestinal tract from side effects associated with anticoagulant therapy by combination an anticoagulant agent and a H2-receptor antagonist (H2RA). These and other features of the present invention are set forth herein more detailed.

In one embodiment, the H2-receptor antagonist (H2RA) is preferably famotidine or nizatidine or ranitidine or an acceptable salt, solvate, hydrate, enantiomer, pro-drug or polymorph thereof.

In one embodiment, the pharmaceutical composition comprises rivaroxaban or a pharmaceutically acceptable salt, solvate, hydrate, enantiomer, pro-drug or polymorph thereof and famotidine or a pharmaceutically acceptable salt, solvate, hydrate, enantiomer, pro-drug or polymorph thereof. In a preferred embodiment, famotidine is present in an amount of 5 mg to 150 mg. In a more preferred embodiment, famotidine is present in an amount of 10 mg to 100 mg.

In one embodiment, the pharmaceutical composition comprises rivaroxaban or a pharmaceutically acceptable salt, solvate, hydrate, enantiomer, pro-drug or polymorph thereof and nizatidine or a pharmaceutically acceptable salt, solvate, hydrate, enantiomer, pro-drug or polymorph thereof. In a preferred embodiment, nizatidine is present in an amount of 10 mg to 500 mg. In a more preferred embodiment, nizatidine is present in an amount of 50 mg to 400 mg.

In one embodiment, the pharmaceutical composition comprises rivaroxaban or a pharmaceutically acceptable salt, solvate, hydrate, enantiomer, pro-drug or polymorph thereof and ranitidine or a pharmaceutically acceptable salt, solvate, hydrate, enantiomer, pro-drug or polymorph thereof. In a preferred embodiment, ranitidine is present in an amount of 5 mg to 500 mg. In a more preferred embodiment, ranitidine is present in an amount of 10 mg to 450 mg.

In a preferred embodiment, rivaroxaban is present in an amount of 1 mg to 250 mg. In a more preferred embodiment, rivaroxaban is present in an amount of 5 mg to 100 mg.

In a preferred embodiment rivaroxaban present in an amount of 5 mg to 100 mg and the famotidine or a pharmaceutically acceptable salt, solvate, hydrate, enantiomer, pro-drug or polymorph thereof is present in an amount of 5 mg to 150 mg.

In a preferred embodiment rivaroxaban present in an amount of 5 mg to 100 mg and the nizatidine or a pharmaceutically acceptable salt, solvate, hydrate, enantiomer, prodrug or polymorph thereof is present in an amount of 10 mg to 500 mg.

In a preferred embodiment rivaroxaban present in an amount of 5 mg to 100 mg and the ranitidine or a pharmaceutically acceptable salt, solvate, hydrate, enantiomer, prodrug or polymorph thereof is present in an amount of 5 mg to 500 mg.

According to main object of the invention, pharmaceutical formulations of rivaroxaban in a combination with a H2-receptor antagonist (H2RA) have a good stability and have improved manufacturing process that is achieved in a technologically simple way according to prior art which overcomes the above described problems.

Yet another object of the present invention is to provide an improved process which is simple, cost-effective and time saving for preparing the pharmaceutical composition of rivaroxaban or pharmaceutically acceptable salts thereof and one or more H2RAs specially.

In another embodiment, the oral formulations described herein can be used to treat, prevent or reduce the risk of almost any physiological disorder for which anticoagulant agents are indicated. The methods and formulations provided herein can be administered to any subject in need of therapy including, without limitation, humans, including patients, companion animals, including but not limited to dogs, cats, ferrets, and birds, food-source animals, including, but not limited to cows, pigs, and sheep, and zoo animals, such as monkeys and other primates, and other similar animal species.

Another embodiment of this invention, the pharmaceutical composition is in the form of solid, liquid or semisolid dosage form.

In one embodiment, these pharmaceutical combinations are administrated oral, parenteral, intranasal, sublingual, transdermal, transmucosal, ophthalmic, intravenous, pulmonary, intramuscular or rectal administration, and preferably for oral administration. According to this embodiment of the invention, said pharmaceutical composition may be formulated with suitable pharmaceutical diluents, excipients or carriers, suitably selected with respect to a dosage form for oral administration. Examples of dosage forms comprise tablets including compressed tablets, coated or uncoated tablets, multilayer tablets, buccal tablets, sublingual tablets, tablet in tablets, in-lay tablets, effervescent compositions, effervescent tablets, immediate release tablets, modified release tablets, ODT-ER (orally disintegrating tablet-extended release), film-coated tablets, orally disintegrating tablets, gastric disintegrating tablets, pills, capsules, hard or soft gelatin capsules, powders, mini tablets, pellets, coated bead systems, granules, microspheres, ion exchange resin systems, sterile solutions or suspensions, sterile ocular solutions, aerosols, sprays, drops, ampoules, suppositories, ocular systems, parenteral systems, creams, gels, ointments, dragees, sachets; films, orally administrable films, solutions, solids; elixirs, tinctures, suspensions, syrups, colloidal dispersions, dispersions, emulsions and thereof.

Another object of the invention is to provide a pharmaceutical composition for use in anticoagulant treatment and treating deep vein thrombosis (DVT) and pulmonary embolism (PE) and protecting the gastrointestinal tract from side effects associated with anticoagulant therapy. Here, H2 receptor antagonist decreases acid level of gastric fluid by inhibiting acid secretion, thereby preventing or minimizing such diseases as gastric erosion, peptic ulcer, major upper gastrointestinal system bleedings, alterations in the intestine permeability and inflammation, all of which are caused by rivaroxaban.

In a preferred embodiment said pharmaceutical composition is formulated preferably in the form of tablet or capsule or multilayer tablet.

In a preferred embodiment said pharmaceutical composition is formulated preferably in the form of tablet or capsule or multilayer tablet comprising rivaroxaban pellets and H2-receptor antagonist (H2RA) pellets.

According to the pharmaceutical incompatibility problem, the present invention provides a pharmaceutical composition comprising a inert barrier layer which is present in the middle in such a way that it separates rivaroxaban and one of the H2-receptor antagonist (H2RA) layers and the rivaroxaban and H2RA layers are present at the outer or inner part of the inert barrier layer. By the virtue of the inert barrier layer, interaction between the rivaroxaban and H2RA molecules is prevented and at the same time H2RA molecule is enabled to remain stable in the dosage form. Moreover, another important reason is that the formulations of the active substances with different release properties can be provided in the same form with inert barrier layer. Thus, rivaroxaban and H2RA molecules are formulated in a way not to interact, and at the same time it is provided that these molecules remain stable in their own form.

In one embodiment of the invention, the pharmaceutical composition may comprise optionally an inert barrier layer between the two molecules wherein the inert barrier layer is selected from the group comprises starch, lactose, sugar alcohol (D-mannitol, erythritol, etc.), lowsubstituted hydroxypropyl cellulose, hydroxypropyl cellulose, hydroxypropyl methylcellulose, polyvinylpyrrolidone, polyvinyl alcohol, methylcellulose, hydroxyethyl methylcellulose or mixtures thereof. In a preferred embodiment of the present invention said barrier layer comprising preferably cellulosic polymers are selected from the group comprises lowsubstituted hydroxypropyl cellulose, hydroxypropyl cellulose, hydroxypropyl methylcellulose, methylcellulose, hydroxyethyl methylcellulose. Thus, the multilayer pellet/tablet according to the present invention comprising the rivaroxaban and H2RA molecules separately and it is characterized in releasing these molecules together and rapidly. The multilayer pellet/tablet according to the present invention is formulated in such a way that H2RA molecules increase the pH of the stomach and after the release of rivaroxaban.

According to the challenges mentioned above the selection of the excipients thus very important. According to this embodiment, one or more pharmaceutically acceptable excipient is selected from the group comprising buffering agents, stabilizers, binders, diluents, dispersing agents, lubricants, glidants, disintegrants, plasticizers, preservatives, sweeteners, flavoring agents, coloring agents or mixtures thereof. Suitable buffering agents may comprise but not limited to alkali metal citrate, citric acid/sodium citrate, tartaric acid, fumaric acid, sorbic acid, citric acid, succinic acid, adipic acid, ascorbic acid, glutaric acid, potassium hydrogen tartrate, sodium hydrogen tartrate, potassium hydrogen phthalate, sodium hydrogen phthalate, potassium dihydrogen phosphate, sodium dihydrogen phosphate, disodium hydrogen phosphate, hydrochloric acid/sodium hydroxide or mixtures thereof, and preferably citric acid, fumaric acid, ascorbic acid, sodium dihydrogen phosphate or mixtures thereof.

Suitable stabilizers may comprise but not limited to citric acid, fumaric acid, tartaric acid, sodium citrate, sodium benzoate, sodium dihydrogen phosphate, calcium carbonate, magnesium carbonate, arginine, lysine, meglamine, tocopherol, butylhydroxyanisole (BHA), butylhydroxytoluene (BHT), ascorbic acid, gallic acid esters or the mixtures thereof, and preferably, citric acid, fumaric acid, arginine or mixtures thereof.

Suitable binders may include but not limited to polyvinylpyrrolidone, polyethylene glycol, polyvinyl alcohol, starch, pregelatinized starch, glucose, glucose syrup, natural gums, sucrose, sodium alginate, cellulose derivatives such as hydroxypropyl methyl cellulose, hydroxypropyl cellulose, carboxy methyl cellulose, methyl cellulose, gelatin, carrageenan, guar gum, carbomer, polymethacrylates, methacrylate polymers, collagens, proteins like gelatin, agar, alginate, alginic acid, xanthan gum, hyaluronic acid, pectin, polysaccharides, carbomer, poloxamer, polyacrylamide, aluminium hydroxide, laponit, bentonit, polyoxyethylene-alkyl ether, polydextrose, polyethylene oxide or mixtures thereof.

Suitable diluents may comprise but not limited to microcrystalline cellulose, mannitol, spray-dried mannitol, lactose, starch, dextrose, sucrose, fructose, maltose, sorbitol, xylitol, inositol, kaolin, inorganic salts, calcium salts, polysaccharides, dicalcium phosphate, sodium chloride, dextrates, lactitol, maltodextrin, sucrose-maltodextrin mixture, trehalose, sodium carbonate, sodium bicarbonate, calcium carbonate or mixtures thereof.

Suitable dispersing agents may comprise but not limited to calcium silicate, magnesium aluminum silicate or mixtures thereof.

Suitable lubricants may comprise but not limited to magnesium stearate, calcium stearate, zinc stearate, talc, waxes, boric acid, hydrogenated vegetable oil, sodium chlorate, magnesium lauryl sulfate, sodium oleate, sodium acetate, sodium benzoate, polyethylene glycol, stearic acid, fatty acid, fumaric acid, glyseryl palmito sulphate, sodium stearyl fumarate, sodium lauryl sulphate or mixtures thereof.

Suitable glidants may comprise but not limited to talc, aluminium silicate, colloidal silica, starch or mixtures thereof.

Suitable disintegrants may comprise but not limited to cross-linked polyvinil pyrrolidone (crospovidone), povidone, cross-linked carboxymethyl cellulose (croscarmellose sodium), low-substituted hydroxypropyl cellulose, pregelatinized starch, sodium carboxymethyl cellulose, calcium carboxymethyl cellulose, carboxymethyl cellulose, docusate sodium, guar gum, low substituted hydroxypropyl cellulose, polyacryline potassium, sodium alginate, corn starch, sodium starch glycolate, alginic acid, alginates, ion-exchange resins, magnesium aluminium silica, sodium dodesyl sulphate, poloxamer, sodium glycine carbonate, sodium lauryl sulphate or mixtures thereof.

Suitable plasticizers may comprise but not limited to polyethylene glycols of different molecular weights, propylene glycol, glycerine, triethyl citrate (TEC), triacetin, diethyl phthalate (DEP), dibutyl phthalate (DBP), tributhyl citrate (TBC), castor oil, dibutyl sebacate (DBS), diacetylated monogglycerides or mixtures thereof.

Suitable preservatives may comprise but not limited to methyl paraben, propyl paraben and their salts (such as sodium, potassium), sodium benzoate, citric acid, benzoic acid, butylated hydroxytoluene, boric acid, sorbic acid, benzyl alcohol, benzalconium chloride, parahydroxybenzoic acids or butylated hydroxyanisole and the like or mixtures thereof.

Suitable sweeteners may comprise but not limited to aspartame, potassium acesulfame, sodium saccharinate, neohesperidine dihydrochalcone, sucralose, saccharin, sugars such as sucrose, glucose, lactose, fructose or sugar alcohols such as mannitol, sorbitol, xylitol, erythritol or mixtures thereof.

Suitable flavoring agents may comprise but not limited to menthol, peppermint, cinnamon, chocolate, vanillin or fruit essences such as cherry, orange, strawberry, grape, black currant, raspberry, banana, red fruits, wild berries or mixtures thereof.

Suitable coloring agents may comprise but not limited to ferric oxide, titanium dioxide, Food, Drug & Cosmetic (FD&C) dyes (such as; FD&C blue, FD&C green, FD&C red, FD&C yellow, FD&C lakes), poncau, indigo Drug & Cosmetic (D&C) blue, indigotine FD&C blue, carmoisine indigotine (indigo Carmine); iron oxides (such as; iron oxide red, yellow, black), quinoline yellow, flaming red, carmine, carmoisine, sunset yellow or mixtures thereof.

In a preferred embodiment of the present invention, said pharmaceutical composition is in the form of tablet or capsule or multilayer tablet comprising rivaroxaban pellets and H2-receptor antagonist (H2RA) pellets which are separate pellets or bilayer or multilayer pellets.

In a preferred embodiment of the present invention said rivaroxaban pellets comprising preferably polyvinyl alcohol (PVA) or isopropyl alcohol (IPA) or sodium alginate or methacrylic acid-ethyl acrylate copolymer (Eudragit EPO) or propylene glycol or lactose monohydrate or sugar pellet or polyvinylpyrrolidone (PVP) mixtures thereof as pharmaceutically acceptable excipients.

In a preferred embodiment of the present invention said H2-receptor antagonist (H2RA) pellets comprising preferably microcrystalline cellulose (MCC) or pregelatinized starch or carbomer or croscarmellose sodium or polyvinylpyrrolidone K30 (PVP K30) or sugar pellet or hydroxypropyl cellulose or coloring agent or mixtures thereof as pharmaceutically acceptable excipients.

According to the pharmaceutical compositions of the invention may be prepared by conventional technology well known to those skilled in the art such as direct compression, dry granulation, wet granulation and the like. During direct compression, active agent and excipients are mixed, sieved and compressed into dosage forms. During wet granulation, the ingredients are mixed and granulated with a granulation liquid. The granulation process provides agglomerates with a desired homogeneity. The mixture is dried and sieved and optionally mixed with additional excipients. Finally, it is compressed into dosage forms. In addition, this novel pharmaceutical formulation is produced by various technologies such as fluidized bed granulation technique or extrusion/spheronization or spray drying and lyofilization.

### Examples:

### Example-1: Rivaroxaban + H2RA tablet or capsule

### Rivaroxaban pellets:

%5.0 - 95 Rivaroxaban
% 0.05 - 20 Polyvinyl alcohol (PVA) or isopropyl alcohol (IPA)
% 0.05 - 20 Sodium alginate
% 0.5 - 40 Methacrylic acid-Ethyl acrylate copolymer (Eudragit EPO) or PVA
%0.01 - 5 Propylene glycol

### H2RA pellets:

%5.0 - 95 Famotidine or Nizatidine or Ranitidine
%5.0- 90 Microcrystalline cellulose (MCC)
%5.0- 90 Pregelatinized starch

### Inert barrier layer:

%0.1- 10 Hydroxypropyl cellulose
%0.1- 10 Yellow iron oxide (coloring agent)

### Other Excipients:

%0.1 - 1.0 Silicon dioxide
%0.1 - 3.0 Magnesium stearate

**Production process of Rivaroxaban pellets:** Alcoholic Methacrylic acid-Ethyl acrylate copolymer (Eudragit EPO) or Polyvinyl alcohol (PVA) solution is sprayed onto rivaroxaban. PVA or IPA (isopropyl alcohol) and propylene glycol solution is sprayed onto this mixture and pellets produced by fluidized bed pelletizing technique. The pellets are coated with sodium alginate in fluidized bed so rivaroxaban pellets are manufactured.

**Production process of H2RA pellets:** Famotidine or Nizatidine or Ranitidine and microcrystalline cellulose and pregelatinized starch are mixed together then by spraying water a wet mass is formed. Pellets produced from this wet mass by extrusion/spheronization pelletizing technique. A solution/dispersion is prepared by adding hydroxypropyl cellulose and yellow iron oxide as inert barrier layer and the H2RA pellets are coated by spraying this mixture.

The rivaroxaban and coated H2RA pellets first mixed with silicon dioxide and then with magnesium stearate. Finally, the pellets are pressed as tablets or filled into the capsules.

### Example-2: Rivaroxaban + H2RA tablet or capsule

### Rivaroxaban pellets:

%5.0 - 95 Rivaroxaban
%0.5 - 40 Methacrylic acid-Ethyl acrylate copolymer (Eudragit EPO) or Polyvinyl alcohol (PVA)
%5.0 - 90 Lactose monohydrate

### H2RA pellets:

%5.0 - 95 Famotidine or Nizatidine or Ranitidine
%5.0- 90 Microcrystalline cellulose (MCC)
%5.0- 90 Pregelatinized starch
%0.1 - 3.0 Carbomer

### Inert barrier layer:

%0.1- 10 Hydroxypropyl cellulose
%0.1- 10 Yellow iron oxide (coloring agent)

### Other Excipients:

%0.1 - 1.0 Silicon dioxide
%0.1 - 3.0 Magnesium stearate

**Production process of Rivaroxaban pellets:** Rivaroxaban and lactose monohydrate are mixed together. Alcoholic Methacrylic acid-Ethyl acrylate copolymer (Eudragit EPO) or Polyvinyl alcohol (PVA) solution is sprayed onto this mixture and pellets produced by fluidized bed pelletizing technique.

**Production process of H2RA pellets:** Famotidine or Nizatidine or Ranitidine and microcrystalline cellulose and pregelatinized starch are mixed together then by spraying carbomer solution a wet mass is formed. Pellets produced from this wet mass by extrusion/spheronization pelletizing technique. A solution/dispersion is prepared by adding hydroxypropyl cellulose and yellow iron oxide as inert barrier layer and he H2RA pellets are coated by spraying this mixture in fluidized bed.

The rivaroxaban and coated H2RA pellets first mixed with silicon dioxide and then with magnesium stearate. Finally, the pellets are pressed as tablets or filled into the capsules.

### Example-3: Coated Core Pelletization (Rivaroxaban pellets +H2RA pellets)

### Rivaroxaban pellets:

%5.0 - 95 Rivaroxaban
%5 - 90 Sugar pellet
%0.1 - 30 Polyvinylpyrrolidone (PVP)
%0.5 - 40 Methacrylic acid-Ethyl acrylate copolymer (Eudragit EPO or Polyvinyl alcohol (PVA)

### H2RA pellets:

%5.0 - 95 Famotidine or Nizatidine or Ranitidine
%5.0- 90 Microcrystalline cellulose (MCC)
%5.0- 40 Croscarmellose sodium
%0.1 - 3.0 Carbomer

### Inert barrier layer:

%0.1- 10 Hydroxypropyl cellulose
%0.1- 10 Yellow iron oxide (coloring agent)

### Other Excipients

%0.1 - 1.0 Silicon dioxide
%0.1 - 3.0 Magnesium stearate

**Production process of Rivaroxaban pellets:** Alcoholic solution/dispersion is prepared by adding rivaroxaban and PVP. Sugar pellets are coated with this solution/dispersion. The sugar pellets which were coated with the active ingredient are coated with methacrylic acid-ethyl acrylate copolymer (Eudragit EPO or Polyvinyl alcohol (PVA)) so rivaroxaban pellets are manufactured.

**Production process of H2RA pellets:** Famotidine or Nizatidine or Ranitidine and microcrystalline cellulose and croscarmellose sodium are mixed together then by spraying carbomer solution a wet mass is formed. Pellets produced from this wet mass by extrusion/spheronization pelletizing technique. A solution/dispersion is prepared by adding hydroxypropyl cellulose and yellow iron oxide as inert barrier layer and the H2RA pellets which were coated with the inert barrier by spraying this mixture.

Coated H2RA pellets and rivaroxaban pellets are mixed. The pellets are first mixed with silicon dioxide and then with magnesium stearate. Finally, the pellets are pressed as tablets or filled into the capsules

### Example-4: Multilayer combination pellet (multilayer pellet production method)

### Multilayer pellets:

### H2RA pellets:

%5.0 - 95 Famotidine or Nizatidine or Ranitidine
%0.1- 30 Polyvinylpyrrolidone K30 (PVP K30)
%5.0 - 90 Sugar pellet
Inert barrier layer (inert coated H2RA pellets):
%0.1- 10 Hydroxypropyl cellulose
%0.1- 30 Polyvinylpyrrolidone K30 (PVP K30)
Rivaroxaban pellets (H2RA pellets are coated with Rivaroxaban):
%15.0 - 60 Rivaroxaban
%0.5 - 40 Methacrylic acid-Ethyl acrylate copolymer (Eudragit EPO) or Polyvinyl alcohol (PVA)
%5.0 - 90 Lactose monohydrate

### Other excipients:

% 0.05 - 20 Polyvinyl alcohol (PVA)
%0.1 - 1.0 Silicon dioxide
%0.1 - 3.0 Magnesium stearate

**Production process multilayer pellets:** A solution/dispersion is prepared by adding Famotidine or Nizatidine or Ranitidine and PVP K30. Sugar pellets are coated with this solution/dispersion. The sugar pellets which were coated with the active ingredient are coated with hydroxypropyl cellulose and PVP K 30 solution/dispersion as inert barrier layer so H2RA pellets are manufactured. Rivaroxaban and lactose monohydrate are mixed with alcoholic Methacrylic acid-Ethyl acrylate copolymer (Eudragit EPO) or Polyvinyl alcohol (PVA) solution. This mixture is sprayed onto H2RA pellets so multilayer pellets are manufactured.

Finally the multilayer pellets are coated by PVA. The multilayer pellets first mixed with silicon dioxide and then with magnesium stearate. The pellets are pressed as tablets or filled into the capsules.

### Example-5: Multilayer combination pellet (multilayer tablet press method)

### Rivaroxaban pellets:

%5.0- 95 Rivaroxaban
%0.5- 40 Methacrylic acid-Ethyl acrylate copolymer (Eudragit EPO) or polyvinyl alcohol (PVA)
%5.0- 90 Lactose monohydrate
% 0.05 - 20 Polyvinyl alcohol (PVA)

### H2RA pellets:

%5.0 - 95 Famotidine or Nizatidine or Ranitidine
%0.1- 30 Polyvinylpyrrolidone K30 (PVP K30)
%5.0 - 90 Sugar pellet
Inert barrier layer (inert coated H2RA pellets):
%0.1 - 10 Hydroxypropyl cellulose
%0.1- 30 Polyvinylpyrrolidone K30 (PVP K30)

### Other excipients:

%0.1 - 1.0 Silicon dioxide
%0.1 - 3.0 Magnesium stearate

**Production process of Rivaroxaban pellets:** Rivaroxaban and lactose monohydrate are mixed together. Alcoholic Methacrylic acid-Ethyl acrylate copolymer (Eudragit EPO) or Polyvinyl alcohol (PVA) solution is sprayed onto this mixture and pellets produced by fluidized bed pelletizing technique.

**Production process of H2RA pellets:** A solution/dispersion is prepared by adding Famotidine or Nizatidine or Ranitidine and PVP K30. Sugar pellets are coated with this solution/dispersion. The sugar pellets which were coated with the active ingredient are coated with hydroxypropyl cellulose and PVP K30 solution/dispersion as inert barrier layer so H2RA pellets are manufactured.

The rivaroxaban and H2RA pellets first mixed with silicon dioxide and then with magnesium stearate. Finally, the pellets are pressed by multilayer rotary tablet press machine. Or dry coating-intertwined tablets which have H2RA pellets on the core are produced with special tablet press machine.

## Claims

1. A pharmaceutical composition comprising rivaroxaban in a combination with a H2-receptor antagonist or a pharmaceutically acceptable salt, solvate, hydrate, enantiomer, pro-drug or polymorph thereof and at least one pharmaceutically acceptable excipient.

2. The pharmaceutical composition according to claim 1, the H2-receptor antagonistis selected from a group comprising bisfentidine, burimamide, cimetidine, dalcotidine, donetidine, ebrotidine, etintidine, famotidine, lafutidine, lamtidine, lavoltidine, loxtidine, lupitidine, metiamide, mifentidine, niperotidine, nizatidine, osutidine, oxmetidine, pibutidine, quisultazine, quisultidine, ramixotidine, ranitidine, ranitidine bismuth citrate, ranitidine hydrochloride, roxatidine, sufotidine, tiotidine, tuvatidine, venritidine, xaltidine or a pharmaceutically acceptable salt, solvate, hydrate, enantiomer, pro-drug or polymorph thereof.

3. The pharmaceutical composition according to claim 2, the H2-receptor antagonists are preferably famotidine or nizatidine or ranitidine or a pharmaceutically acceptable salt, solvate, hydrate, enantiomer, pro-drug or polymorph thereof.

4. The pharmaceutical composition according to claim 1 or 3, wherein said pharmaceutical composition comprising rivaroxaban and famotidine or a pharmaceutically acceptable salt, solvate, hydrate, enantiomer, pro-drug or polymorph thereof.

5. The pharmaceutical composition according to claim 1 or 3, wherein said pharmaceutical composition comprising rivaroxaban and nizatidine or a pharmaceutically acceptable salt, solvate, hydrate, enantiomer, pro-drug or polymorph thereof.

6. The pharmaceutical composition according to claim 1 or 3, wherein said pharmaceutical composition comprising rivaroxaban and ranitidine or a pharmaceutically acceptable salt, solvate, hydrate, enantiomer, pro-drug or polymorph thereof.

7. The pharmaceutical composition according to claim 1, wherein rivaroxaban is present in an amount of 1 mg to 250 mg.

8. The pharmaceutical composition according to claim 7, wherein rivaroxaban is preferably in an amount of 5 mg to 100 mg.

9. The pharmaceutical composition according to claim 4 or 8, wherein rivaroxaban present in an amount of 5 mg to 100 mg and famotidine or a pharmaceutically acceptable salt, solvate, hydrate, enantiomer, pro-drug or polymorph thereof is present in an amount of 5 mg to 150 mg.

10. The pharmaceutical composition according to claim 5 or 8, wherein rivaroxaban present in an amount of 5 mg to 100 mg and nizatidine or a pharmaceutically acceptable salt, solvate, hydrate, enantiomer, pro-drug or polymorph thereof is present in an amount of 10 mg to 500 mg.

11. The pharmaceutical composition according to claim 6 or 8, wherein rivaroxaban present in an amount of 5 mg to 100 mg and ranitidine or a pharmaceutically acceptable salt, solvate, hydrate, enantiomer, pro-drug or polymorph thereof is present in an amount of 5 mg to 500 mg.

12. The pharmaceutical composition according to claim 1, wherein said pharmaceutical composition is in the form of solid, liquid or semisolid dosage form.

13. The pharmaceutical composition according to any preceding claims, wherein said pharmaceutical composition is formulated as tablets comprising compressed tablets, coated or uncoated tablets, multilayer tablets, buccal tablets, sublingual tablets, tablet in tablets, in-lay tablets, effervescent compositions, effervescent tablets, immediate release tablets, modified release tablets, ODT-ER (orally disintegrating tablet-extended release), film-coated tablets, orally disintegrating tablets, gastric disintegrating tablets, pills, capsules, hard or soft gelatin capsules, powders, mini tablets, pellets, coated bead systems, granules, microspheres, ion exchange resin systems, sterile solutions or suspensions, sterile ocular solutions, aerosols, sprays, drops, ampoules, suppositories, ocular systems, parenteral systems, creams, gels, ointments, dragees, sachets; films, orally administrable films, solutions, solids; elixirs, tinctures, suspensions, syrups, colloidal dispersions, dispersions, emulsions.

14. The pharmaceutical composition according to claim 13, wherein said pharmaceutical composition is formulated preferably in the form of tablet or capsule or multilayer tablet.

15. The pharmaceutical composition according to claim 14, wherein said pharmaceutical composition is formulated preferably in the form of tablet or capsule or multilayer tablet comprising rivaroxaban pellets and H2-receptor antagonist pellets.

16. The pharmaceutical composition according to claim 14 or 15, further comprising an inert barrier layer.

17. The pharmaceutical composition according to claim 16, wherein said inert barrier layer is selected from the group comprising starch, lactose, sugar alcohol, lowsubstituted hydroxypropyl cellulose, hydroxypropyl cellulose, hydroxypropyl methylcellulose, polyvinylpyrrolidone, polyvinyl alcohol, methylcellulose, hydroxyethyl methylcellulose or mixtures thereof.

18. The pharmaceutical composition according to claim 17, wherein said barrier layer comprising preferably cellulosic polymers.

19. The pharmaceutical composition according to any preceding claims, wherein the one or more pharmaceutically acceptable excipients are selected from the group comprising buffering agents, stabilizers, binders, diluents, dispersing agents, lubricants, glidants, disintegrants, plasticizers, preservatives, sweeteners, flavoring agents, coloring agents or mixtures thereof.

20. The pharmaceutical composition according to claim 13 or 14, wherein said pharmaceutical composition is in the form of tablet or capsule or multilayer tablet comprising rivaroxaban pellets and H2-receptor antagonist pellets which are separate pellets or bilayer or multilayer pellets.

21. The pharmaceutical composition according to claim 20, wherein said rivaroxaban pellets comprising preferably polyvinyl alcohol or isopropyl alcohol or sodium alginate or methacrylic acid-ethyl acrylate copolymer or propylene glycol or lactose monohydrate or sugar pellet or polyvinylpyrrolidone mixtures thereof

22. The pharmaceutical composition according to claim 20, wherein said H2-receptor antagonist pellets comprising preferably microcrystalline cellulose or pregelatinized starch or carbomer or croscarmellose sodium or polyvinylpyrrolidone K30 or sugar pellet or hydroxypropyl cellulose or coloring agent or mixtures thereof.

23. The pharmaceutical composition of any preceding claims, for use in anticoagulant treatment and treating deep vein thrombosis and pulmonary embolism and protecting the gastrointestinal tract from side effects associated with anticoagulant therapy.
